# EUROPEAN PATENT APPLICATION

(11) **EP 4 769 415 A1**
(43) Date of publication of application: **01.07.2026**
(21) Application number: 24858526.7
(22) Date of filing: 26.08.2024
(51) Int. Cl.: G16B 5/00

(54) **DIGITAL STANDARD FOR VERIFYING ACCURACY OF METAGENOME BIOLOGICAL INFORMATION DETECTION**

(30) Priority: 25.08.2023 CN 202311081718
(71) Applicant: Huzhou Shenke Biotechnology Co., Ltd., Huzhou, Zhejiang 313000 (CN)
(72) Inventor: ZENG, Dewei, Huzhou, Zhejiang 313001 (CN); YANG, Zhixing, Huzhou, Zhejiang 313001 (CN); MA, Quangang, Huzhou, Zhejiang 313001 (CN); WANG, Yueli, Huzhou, Zhejiang 313001 (CN)
(74) Representative: Mewburn Ellis LLP
(86) International application number: PCT/CN2024/114568
(87) International publication number: WO 2025/044990

(57) **Abstract**

Provided in the present invention is a digital standard for verifying the accuracy of the metagenome biological information detection process. In the present invention, 24 viral genomes of particular concern to the Chinese Pharmacopoeia are selected, and a total of 100 samples are constructed. Each sample contains 24 different viral genome sequences. The abundance is randomly generated, and the viral abundance distribution of each sample is different. The digital standard of the present invention can be used for verifying the accuracy of the biological information process built on the basis of metagenome.

## Description

### Technical field

The present invention relates to the field of biological information. In particular, the present invention relates to digital standards for verifying the accuracy of metagenomic biological information detection.

### Background

In metagenomics, total DNAs of all microorganisms from a microecological sample are extracted to construct a metagenomic library, and then the genetic composition of all microorganisms in the sample and the potential functions of their communities are analyzed by using genomics research strategies. This technology does not rely on the isolation and pure culture techniques of single bacteria, which largely solves the problem that most microorganisms are difficult to be studied since they cannot be isolated and cultured. At the same time, the real situation of the microbial composition in the studied ecological environment can also be reflected. In microecological research related to human health, the quantification of microorganisms based on metagenomic sequencing data is the basis for studying their community composition, interspecies interactions, and exploring the relationship between them and the occurrence and development of diseases, as well as other related rules. With the progress of scientific research, more and more studies have shown that it is increasingly important to accurately annotate lower-level taxonomic units of specific species, namely strains. If the relationship between bacteria and diseases is simply studied at a higher taxonomic level, it is likely to group together categories that are positively correlated, irrelevant, or even negatively correlated with the development of diseases, which has obvious fallacies both biologically and statistically. Moreover, it is urgently needed to correct existing research results by improving the accuracy of microbial quantification or conducting more in-depth mechanistic research.

In the current testing industry of biological products, more and more people use metagenomic technology for exogenous virus detection. Through high-throughput metagenomic technology, it is expected to detect all microorganisms in a sample at once (bacteria, viruses, fungi, etc.), but its subsequent verification work needs to be carried out as soon as possible to popularize this detection technology.

Metagenomics technology developed in recent years avoids traditional microbial isolation and culture methods, directly extracts total DNAs from environmental samples, and obtains new functional genes and bioactive substances by constructing and screening metagenomic libraries. Metagenomic libraries include the genetic information of both culturable and unculturable microorganisms, thus increasing the chance of obtaining new bioactive substances. The resolution of the quantitative methods is relatively high, which can annotate to the species or strain level, so that it is currently widely used in pathogen detection.

In the field of biological product testing, currently, traditional methods are still mainly used for testing, such as PCR, electrophoresis, and culture methods. However, these traditional methods exhibit low throughput and cannot simultaneously handle a large number of different types of viruses. Therefore, methods based on next-generation sequencing (NGS) technology are gradually developing.

Although next-generation sequencing can make up for the disadvantage of low throughput, its characteristics such as high sensitivity, high background noise, and sequencing randomness make it difficult to judge the true and false positives of the detection results. At the same time, sample preparation for next-generation sequencing, extraction of DNAs and RNAs, and subsequent library construction processes are prone to introduce contamination from the environment or consumables. Therefore, while promoting the use of metagenomics for safety testing of biological products, it is necessary to develop reference materials to test the entire metagenomics workflow, including dry experiments and wet experiments. Among them, the methods and tools for dry experiments (bioinformatics processes) are varied, and there is currently no generally recognized and most reliable detection method and tool to be used for everyone. Therefore, it is necessary to construct digital standards to verify bioinformatics analysis processes.

Therefore, there is a need in this field to develop a digital standard for verifying the accuracy of metagenomic bioinformatics detection.

### Summary of the invention

The purpose of the present invention is to provide a digital standard for verifying the accuracy of metagenomic bioinformatics detection.

In the first aspect of the present invention, a method for preparing a digital standard for genomic bioinformation detection is provided, which includes following steps:
(i) providing reference genomes of n microorganisms, where n is an integer selected from 10-24, preferably 15-24, and more preferably 20-24;
(ii) simulated-community design: generating a simulated community based on the reference genome and classification information;
(iii) reading simulation: using a read simulator to sample from each genome of the simulated community to generate simulated samples, and mixing the simulated samples to obtain a simulated dataset;
(iv) standard assembly: assembling the simulated dataset to obtain the digital standard.

In another preferred embodiment, the microorganism is a virus.

In another preferred embodiment, the microorganism is n microorganisms selected from the group consisting of: *Betapolyomavirus macacae* (Simian Virus 40), *Bluetongue virus*, *Bovine polyomavirus 3*, *Human betaherpesvirus 6A* (Human betaherpesvirus-6), *Human betaherpesvirus 6B* (Human betaherpesvirus-6), *Fowl aviadenovirus A* (Exogenous Avian Adenovirus Type I), *Simian retrovirus 8* (Simian retrovirus), *Simian foamy virus*, *Human gammaherpesvirus 4* (Human Epstein-Barr virus) (Epstein-Barr virus), *Human polyomavirus 12* (Human polyomavirus), *Human circovirus VS6600022* (Human circovirus), *Human betaherpesvirus 5* (Human Cytomegalovirus), *Human immunodeficiency virus 1* (Human immunodeficiency virus), *Murine respirovirus* (Sendai Virus), *Murid betaherpesvirus 8* (Murine Salivary Gland Virus_Murine Cytomegalovirus), *Nipah henipavirus*, *Suid alphaherpesvirus 1* (Pseudorabies Virus), *Swinepox virus*, *Porcine reproductive and respiratory syndrome virus*, *Suid betaherpesvirus 2* (Porcine Cytomegalovirus), *Porcine circovirus 2*, *Human gammaherpesvirus 8* (Human Herpesvirus-8), *Bat rotavirus* (Mouse rotavirus), *Murid betaherpesvirus 1* (Murine Salivary Gland Virus_Murine Cytomegalovirus).

In another preferred embodiment, the n microorganisms consist of: *Betapolyomavirus macacae* (Simian Virus 40), *Bluetongue virus*, *Bovine polyomavirus 3*, *Human betaherpesvirus 6A* (Human betaherpesvirus-6), *Human betaherpesvirus 6B* (Human betaherpesvirus-6), *Fowl aviadenovirus A* (Exogenous Avian Adenovirus Type I), *Simian retrovirus 8* (Simian retrovirus), *Simian foamy virus*, *Human gammaherpesvirus 4* (Human Epstein-Barr virus) (Epstein-Barr virus), *Human polyomavirus 12* (Human polyomavirus), *Human circovirus VS6600022* (Human circovirus), *Human betaherpesvirus 5* (Human Cytomegalovirus), *Human immunodeficiency virus 1* (Human immunodeficiency virus), *Murine respirovirus* (Sendai Virus), *Murid betaherpesvirus 8* (Murine Salivary Gland Virus_Murine Cytomegalovirus), *Nipah henipavirus*, *Suid alphaherpesvirus 1* (Pseudorabies Virus), *Swinepox virus*, *Porcine reproductive and respiratory syndrome virus*, *Suid betaherpesvirus 2* (Porcine Cytomegalovirus), *Porcine circovirus 2*, *Human gammaherpesvirus 8* (Human Herpesvirus-8), *Bat rotavirus* (Mouse rotavirus), *Murid betaherpesvirus 1* (Murine Salivary Gland Virus_Murine Cytomegalovirus).

In another preferred embodiment, the reference genomes are obtained from the NCBI Genome Database.

In another preferred embodiment, the steps (ii) to (iv) are performed using CAMISIM software.

In another preferred embodiment, the simulated dataset described in step (ii) is generated by a method selected from the group consisting of de novo simulation, dataset simulation, or a combination thereof.

In another preferred embodiment, the de novo simulation includes following steps:
A1) providing a specified taxonomic information, including the taxonomic unit names and taxonomic unit abundance;
A2) providing the genome classification names of the reference genomes in known databases, and mapping the taxonomic unit names to the genome classification names in the known databases (such as NCBI database classification IDs), thereby matching the taxonomic units with the reference genomes;
A3) based on the novelty level of the reference genomes and the corresponding taxonomic unit names, extracting m genomes from the reference genomes to generate the simulated community.

In another preferred embodiment, the specified taxonomic information is a CAMI file or a BIOM file.

In another preferred embodiment, the species taxonomic unit names refer to the operational taxonomic unit (OTU) classification names.

In another preferred embodiment, in step A2), the OUT classification names are mapped to the NCBI taxonomic IDs.

In another preferred embodiment, in step A2), the taxonomic unit and the mapped genome satisfy following conditions:
the taxonomic unit name and the genome classification name have the minimum path calculated based on the de Bruijn graph, and
the taxonomic unit name and the genome classification name belong to at least the same family.

In another preferred embodiment, in step A2), if there is no genome that meets the conditions, the taxonomic unit is assigned to a custom new "random" genome.

In another preferred embodiment, step A3) includes following steps:
1) based on the total number of genomes m, extracting the same number of genomes from each novelty category (new strain, new species, new genus, new family, new order);
2) if there are not enough genomes available for being extracted in a certain novelty category, extracting more genomes in equal amounts from other categories;
3) repeating steps 1) and 2) until m genomes have been extracted.

In another preferred embodiment, the novelty category is determined based on the degree of similarity between the reference genome sequence and the currently known viral sequences.

In another preferred embodiment, the value of m is 15-40, preferably 20-25.

In another preferred embodiment, the simulated community also contains simulated additional genomes in steps, and the additional genomes are generated through following steps:
a) selecting a specific genome from the reference genome, and extracting the strain number from the geometric distribution, where the strain number represents the number of related strains created for the specific genome;
b) repeating this step until the specified total number of strains is reached;
c) from the generated related strains, randomly selecting the specified strain number to obtain the simulated additional genomes.

In another preferred embodiment, for each reference genome, 20-50 related strains, preferably 30-40 related strains are generated.

In another preferred embodiment, the genetic distance between each of the related strains and the starting strain increases stepwise with a step size of 0.1%, with a total distance of 2%-5%, preferably 3%-4%.

In another preferred embodiment, the dataset simulation includes following steps:
B1) sampling from a log-normal distribution to create a set of abundance distribution samples;
B2) generating the simulated community based on the reference genome and the set of abundance distribution samples.

In another preferred embodiment, in step B1), the default data of the CAMISIM software is used, and the parameters of the log-normal distribution are set to mu = 1 and sigma = 2.

In another preferred embodiment, in step B1), ≥ 2, preferably ≥ 5, more preferably ≥ 10 abundance distribution samples are independently extracted from the log-normal distribution using the same parameter settings.

In another preferred embodiment, in step B1), for the (x+1)^{th} abundance distribution sample, a new value is obtained by sampling from the log-normal distribution, and this new value is added to the value of the x^{th} abundance distribution sample and divided by 2 to obtain the (x+1)^{th} abundance distribution sample.

In another preferred embodiment, in step (iii), simulated reads are generated according to specified parameters, which include: read length, insert size, error rate, or a combination thereof.

In another preferred embodiment, the read length is 50-500 bp, preferably 100-350 bp, and more preferably 150-250 bp.

In another preferred embodiment, the insert size is 120 bp-400 bp, preferably 220 bp-320 bp, and more preferably 270 bp-280 bp.

In another preferred embodiment, the error rate is ≤ 10%, preferably ≤ 5%, and more preferably ≤ 1%.

In another preferred embodiment, in step (iii), the sampling is performed proportionally from each genome of the simulated community according to the specified genome abundance.

In another preferred embodiment, the simulated dataset contains 10-500 simulated samples, preferably 50-200 simulated samples, and more preferably 100-150 simulated samples.

In another preferred embodiment, the specified abundances in each of the samples are different.

In another preferred embodiment, step (iii) further includes a step of: using the same genomic abundance and different average insertion sizes to generate read simulations for the second time, and mixing them with the previously obtained simulated samples.

In another preferred embodiment, in step (iii), the size of each sample in the simulated dataset contains 1-10G simulated reads, preferably 3-5G.

In another preferred embodiment, in step (iii), the generated simulated dataset includes, for each sample:
simulated read sequences, such as FASTQ files; and
alignment information between the simulated reads and the reference genome, such as BAM files.

In another preferred embodiment, step (iv) includes following steps:
iva) obtaining the position of each read on the reference genome from the read simulation, and calculating the coverage of each locus;
ivb) decomposing the reference genome sequence at zero-coverage loci to obtain the gold standard contig sequences.

In another preferred embodiment, step (iv) includes the following step:
ivc) shuffling and anonymizing the sequences of the simulated dataset to obtain the challenge dataset.

In another preferred embodiment, the method further includes step (v) after step (iv): filtering the data according to the base and sequence quality of the sequencing data of the digital standard; and using sequence alignment software to compare and identify the digital standard with a database built based on the reference genome, and remove the sequences that are aligned to non-target viral genomes.

In another preferred embodiment, the digital standard contains the reference genome species contained in each simulated sample and the corresponding abundance information.

In the second aspect of the present invention, a digital standard for metagenomic bioinformatics detection is provided. The digital standard comprises p samples, wherein each sample contains genomic sequences from reference genomes of n different microorganisms,
and the p samples have different genomic abundances.

In another preferred embodiment, the microorganism is a virus.

In another preferred embodiment, the microorganism is n microorganisms selected from the group consisting of: *Betapolyomavirus macacae* (Simian Virus 40), *Bluetongue virus*, *Bovine polyomavirus 3*, *Human betaherpesvirus 6A* (Human betaherpesvirus-6), *Human betaherpesvirus 6B* (Human betaherpesvirus-6), *Fowl aviadenovirus A* (Exogenous Avian Adenovirus Type I), *Simian retrovirus 8* (Simian retrovirus), *Simian foamy virus*, *Human gammaherpesvirus 4* (Human Epstein-Barr virus) (Epstein-Barr virus), *Human polyomavirus 12* (Human polyomavirus), *Human circovirus VS6600022* (Human circovirus), *Human betaherpesvirus 5* (Human Cytomegalovirus), *Human immunodeficiency virus 1* (Human immunodeficiency virus), *Murine respirovirus* (Sendai Virus), *Murid betaherpesvirus 8* (Murine Salivary Gland Virus_Murine Cytomegalovirus), *Nipah henipavirus*, *Suid alphaherpesvirus 1* (Pseudorabies Virus), *Swinepox virus*, *Porcine reproductive and respiratory syndrome virus*, *Suid betaherpesvirus 2* (Porcine Cytomegalovirus), *Porcine circovirus 2*, *Human gammaherpesvirus 8* (Human Herpesvirus-8), *Bat rotavirus* (Mouse rotavirus), *Murid betaherpesvirus 1* (Murine Salivary Gland Virus_Murine Cytomegalovirus).

In another preferred embodiment, the n microorganisms consist of: *Betapolyomavirus macacae* (Simian Virus 40), *Bluetongue virus*, *Bovine polyomavirus 3*, *Human betaherpesvirus 6A* (Human betaherpesvirus-6), *Human betaherpesvirus 6B* (Human betaherpesvirus-6), *Fowl aviadenovirus A* (Exogenous Avian Adenovirus Type I), *Simian retrovirus 8* (Simian retrovirus), *Simian foamy virus*, *Human gammaherpesvirus 4* (Human Epstein-Barr virus) (Epstein-Barr virus), *Human polyomavirus 12* (Human polyomavirus), *Human circovirus VS6600022* (Human circovirus), *Human betaherpesvirus 5* (Human Cytomegalovirus), *Human immunodeficiency virus 1* (Human immunodeficiency virus), *Murine respirovirus* (Sendai Virus), *Murid betaherpesvirus 8* (Murine Salivary Gland Virus_Murine Cytomegalovirus), *Nipah henipavirus*, *Suid alphaherpesvirus 1* (Pseudorabies Virus), *Swinepox virus*, *Porcine reproductive and respiratory syndrome virus*, *Suid betaherpesvirus 2* (Porcine Cytomegalovirus), *Porcine circovirus 2*, *Human gammaherpesvirus 8* (Human Herpesvirus-8), *Bat rotavirus* (Mouse rotavirus), *Murid betaherpesvirus 1* (Murine Salivary Gland Virus_Murine Cytomegalovirus).

In another preferred embodiment, p is an integer selected from 10-500, preferably 50-200, and more preferably 100-150.

In another preferred embodiment, n is an integer selected from 10-24, preferably 15-24, and more preferably 20-24.

In another preferred embodiment, the digital standard contains information on the reference genome species contained in each sample and their corresponding abundance.

In another preferred embodiment, the digital standard is filtered. The filtering refers to align and identify the digital standard with a database built based on the reference genome by using sequence alignment software, and removing sequences that align to non-target viral genomes.

In another preferred embodiment, the digital standard is constructed by the method described in the first aspect of the present invention.

In the third aspect of the present invention, a device for preparing a digital standard for metagenomic bioinformatics detection is provided, and the device includes:
1) an input module, which is used to input reference genomes of n microorganisms, wherein n is an integer selected from 10-24, preferably 15-24, and more preferably 20-24;
2) a simulation module, which is used to:
   generate a simulated community based on the reference genomes and classification information;
   sample from each genome of the simulated community by using a read simulator to generate simulated samples, and mix the simulated samples to obtain a simulated dataset;
   assemble the simulated dataset to obtain the digital standard; and
3) an output module, which is used to output the digital standard.

In another preferred embodiment, the microorganism is a virus.

In another preferred embodiment, the microorganism is n microorganisms selected from the group consisting of: *Betapolyomavirus macacae* (Simian Virus 40), *Bluetongue virus*, *Bovine polyomavirus 3*, *Human betaherpesvirus 6A* (Human betaherpesvirus-6), *Human betaherpesvirus 6B* (Human betaherpesvirus-6), *Fowl aviadenovirus A* (Exogenous Avian Adenovirus Type I), *Simian retrovirus 8* (Simian retrovirus), *Simian foamy virus*, *Human gammaherpesvirus 4* (Human Epstein-Barr virus) (Epstein-Barr virus), *Human polyomavirus 12* (Human polyomavirus), *Human circovirus VS6600022* (Human circovirus), *Human betaherpesvirus 5* (Human Cytomegalovirus), *Human immunodeficiency virus 1* (Human immunodeficiency virus), *Murine respirovirus* (Sendai Virus), *Murid betaherpesvirus 8* (Murine Salivary Gland Virus_Murine Cytomegalovirus), *Nipah henipavirus*, *Suid alphaherpesvirus 1* (Pseudorabies Virus), *Swinepox virus*, *Porcine reproductive and respiratory syndrome virus*, *Suid betaherpesvirus 2* (Porcine Cytomegalovirus), *Porcine circovirus 2*, *Human gammaherpesvirus 8* (Human Herpesvirus-8), *Bat rotavirus* (Mouse rotavirus), *Murid betaherpesvirus 1* (Murine Salivary Gland Virus_Murine Cytomegalovirus).

In another preferred embodiment, the n microorganisms consist of: *Betapolyomavirus macacae* (Simian Virus 40), *Bluetongue virus*, *Bovine polyomavirus 3*, *Human betaherpesvirus 6A* (Human betaherpesvirus-6), *Human betaherpesvirus 6B* (Human betaherpesvirus-6), *Fowl aviadenovirus A* (Exogenous Avian Adenovirus Type I), *Simian retrovirus 8* (Simian retrovirus), *Simian foamy virus*, *Human gammaherpesvirus 4* (Human Epstein-Barr virus) (Epstein-Barr virus), *Human polyomavirus 12* (Human polyomavirus), *Human circovirus VS6600022* (Human circovirus), *Human betaherpesvirus 5* (Human Cytomegalovirus), *Human immunodeficiency virus 1* (Human immunodeficiency virus), *Murine respirovirus* (Sendai Virus), *Murid betaherpesvirus 8* (Murine Salivary Gland Virus_Murine Cytomegalovirus), *Nipah henipavirus*, *Suid alphaherpesvirus 1* (Pseudorabies Virus), *Swinepox virus*, *Porcine reproductive and respiratory syndrome virus*, *Suid betaherpesvirus 2* (Porcine Cytomegalovirus), *Porcine circovirus 2*, *Human gammaherpesvirus 8* (Human Herpesvirus-8), *Bat rotavirus* (Mouse rotavirus), *Murid betaherpesvirus 1* (Murine Salivary Gland Virus_Murine Cytomegalovirus).

In the fourth aspect of the present invention, a method for verifying the accuracy of metagenomic bioinformatics detection is provided, which includes following steps:
I) providing a metagenomic bioinformatics detection method to be tested;
II) using the detection method to detect each sample in the digital standard according to the second aspect of the present invention, so as to obtain the detection results of the detection method;
III) comparing the detection results of the detection method with the reference genome species and corresponding abundance information of each sample in the digital standard, so as to judge the accuracy of the detection method.

In another preferred embodiment, the method includes comparing whether the abundance information of the same virus in the standard is consistent with the detection result of the biological information detection method to be tested.

In the fifth aspect of the present invention, a device for verifying the accuracy of a metagenomic bioinformatics detection method is provided, and the device includes:
I) an input module, which is used for inputting the metagenomic bioinformatics detection method to be tested;
II) a calculation module, which uses the detection method to detect the digital standard according to the second aspect of the present invention, so as to obtain the detection result of the method to be tested;
III) a comparison module, which compares the detection result of the detection method with the reference genome species and corresponding abundance information of each sample in the digital standard, so as to judge the accuracy of the bioinformatics detection method;
IV) an output module, which is used for outputting the accuracy judgment result of the bioinformatics detection method.

It should be understood that within the scope of the present invention, the various technical features of the present invention described above and the various technical features specifically described below (such as in the examples) can be combined with each other to form new or preferred technical solutions, which will not be repeated herein one by one due to the limited contents.

### Description of drawings

Following drawings are used to illustrate the specific embodiments of the present invention, and are not used to limit the scope of the present invention which is defined by the claims.
Figure 1 shows the flow chart of constructing simulated data for specific reference genomes using CAMISIM.
Figure 2 shows an example of the results of verifying the accuracy of bioinformatics detection and comparison using the digital standard.
Figure 3 shows the overlapping results of virus species between the standards constructed using 59 viruses as digital standards and the detection results.
Figure 4 shows an example of the results of verifying the accuracy of bioinformatics detection and comparison of the standards constructed using 59 viruses as digital standards.

### Modes for carrying out the invention

After an extensive and in-depth research, the inventors have developed, for the first time, a digital standard for verifying the accuracy of metagenomic bioinformatics detection workflows. Specifically, in the present invention, 24 viral genomes of particular concern in the Chinese Pharmacopoeia were selected to construct a digital standard that can be used to verify the accuracy of metagenomic bioinformatics detection workflows, including both qualitative and quantitative aspects. In the present invention, a total of 100 samples were constructed, each containing genomic sequences of 24 different viruses with randomly generated abundances, and the viral abundance distribution of each sample is different. Through this batch of digital standards, the accuracy of bioinformatics workflows built based on metagenomics can be effectively verified. On this basis, the present invention has been completed.

### Standard

The digital standard of the present invention is constructed by the method described in the first aspect of the present invention. Specifically, it is constructed based on the known viral genomes selected from Table 1 below:

**Table 1 Viral genome information**

| **NCBI_ID** | **Virus English name** | **Genome length (MB)** |
|---|---|---|
| 1891767 | *Betapolyomavirus macacae* | 0.005243 |
| 40051 | *Bluetongue virus* | 0.019179 |
| 1561705 | *Bovine polyomavirus 3* | 0.004861 |
| 32603 | *Human betaherpesvirus 6A* | 0.159378 |
| 32604 | *Human betaherpesvirus 6B* | 0.162114 |
| 190061 | *Fowl aviadenovirus A* | 0.043807 |
| 1904439 | *Simian retrovirus 8* | 0.008126 |
| 11642 | *Simian foamy virus* | 0.012965 |
| 10376 | *Human gammaherpesvirus 4* (*Epstein-Barr virus*) | 0.171896 |
| 1303334 | *Human polyomavirus 12* | 0.005033 |
| 1525173 | *Human circovirus VS6600022* | 0.002836 |
| 10359 | *Human betaherpesvirus 5* | 0.234127 |
| 11676 | *Human immunodeficiency virus 1* | 0.008955 |
| 11191 | *Murine respirovirus* | 0.015384 |
| 1261657 | *Murid betaherpesvirus 8* | 0.202949 |
| 121791 | *Nipah henipavirus* | 0.018 |
| 10345 | *Suid alphaherpesvirus 1* | 0.142922 |
| 10276 | *Swinepox virus* | 0.146454 |
| 28344 | *Porcine reproductive and respiratory syndrome virus* | 0.015428 |
| 1608255 | *Suid betaherpesvirus 2* | 0.128367 |
| 85708 | *Porcine circovirus 2* | 0.001767 |
| 37296 | *Human gammaherpesvirus 8* | 0.132644 |
| 1340801 | *Bat rotavirus* | 0.017637 |
| 10366 | *Murid betaherpesvirus 1* | 0.230343 |

The size of a viral genome is at least several kilobases to tens of kilobases. The base difference between a viral subtype and the original type may be only 100-200 bases, which accounts for a very small proportion of the entire genome. Since the read length of next-generation sequencing is usually 250-300 bp, it is difficult to ensure that sequences containing different bases can be selected during the random library construction process. Current viral alignment methods still rely on sequencing accuracy, and generally, one base mismatch is allowed in principle. However, when distinguishing subtypes, a single-base difference may represent different viral subtypes, thereby leading to incorrect alignment of the final sequencing results. Moreover, most sequences among viruses in the current virus database are relatively conserved, and there is no sequence database that can completely distinguish viral subtypes.

In the present invention, the viruses of concern in the Chinese Pharmacopoeia were selected. When screening approximately 100 viruses of concern in the Chinese Pharmacopoeia, it was found that since they contain many viral subtypes and their genomes contain highly similar sequences of viral subtypes, the prepared digital standards often contain a large number of similar sequences. Using such digital standards to evaluate the accuracy of bioinformatics detection methods will cause interference, making it difficult to obtain true and reliable evaluation results.

After extensive attempts and screening, 24 types of viruses as shown in Table 1 were selected in the present invention. These viruses have a wide range of sources, including those from cattle, mice, humans, and pigs. Moreover, they contain no or basically no similar viral sequences, so that they will not interfere with the test results of digital standards and can be accurately used to evaluate bioinformatic detection methods. At the same time, all these viruses are viruses of concern in the Chinese Pharmacopoeia, and the digital standards composed of them have extremely high clinical significance and application value.

### CAMISIM

The method for preparing the digital standard of the present invention is carried out using microbial communities and the metagenome simulator CAMISIM. The CAMISIM software can simulate different microbial abundance profiles, multi-sample time series, and differential abundance studies, including real and simulated strain-level diversity, and generate second-generation and third-generation sequencing data.

CAMISIM allows a custom-generation of many attributes of communities and datasets, such as the total number of genomes, species diversity, genome abundance distribution, sample size, number of replicates, and sequencing technology used. The sequencing data simulated by the CAMISIM software is highly consistent with real data. Based on existing technologies, results highly consistent with real data were observed in two sets of simulated multi-sample data of human and mouse gut microbial communities generated by the CAMISIM software. Meanwhile, impacts of different genome divergences, sequencing depths, and read errors on two popular metagenomic sequence assembly software programs, MEGAHIT and metaSPAdes, were studied using thousands of small datasets generated by CAMISIM.

The data simulation of CAMISIM is divided into three stages:
1) Community design, which includes selecting community members and their genomes, and assigning their relative abundances; wherein genome selection is based on the truncated geometric distribution, and abundance is based on the lognormal distribution;
2) Metagenomic sequencing data simulation;
3) Post-processing, including binning and assembly steps.

### Kraken2

In one embodiment, during the preparation process of the digital standard of the present invention, alignment with known sequences can be performed to remove simulated sequences of non-target viral genomes, thereby improving the accuracy of the final verification. This alignment can be carried out using Kraken software.

Kraken and Kraken2 (an iterative version of Kraken) are the most commonly used software in microbiome analysis. Compared with other software with the same function, they are characterized by high speed and high accuracy,the principle of which is a high-precision metagenomic sequence classification software based on the k-mer algorithm, which can quickly classify sequenced (or simulated) reads into species.

There are two steps for using Kraken software: preparation (database construction) and identification.

### Step 1: Preparation (Database Construction)

- constructing a taxon database corresponding to k-mer (Box1)
- mapping the database and index files to the memory

### Step 2: Identification

- cutting the sequence to be identified into k-mers
- aligning the k-mers to the database to obtain their LCA_taxon (Box2) and the number of alignments.
- constructing the above data into a classification tree, then calculating the sum of all weights on each root-to-leaf path, so that the maximum is the classification tree of that sequence.

### Method for constructing digital standard

The method for constructing the digital standard of the present invention requires: 1) the CAMISIM tool, 2) reference genomes of specific microorganisms.

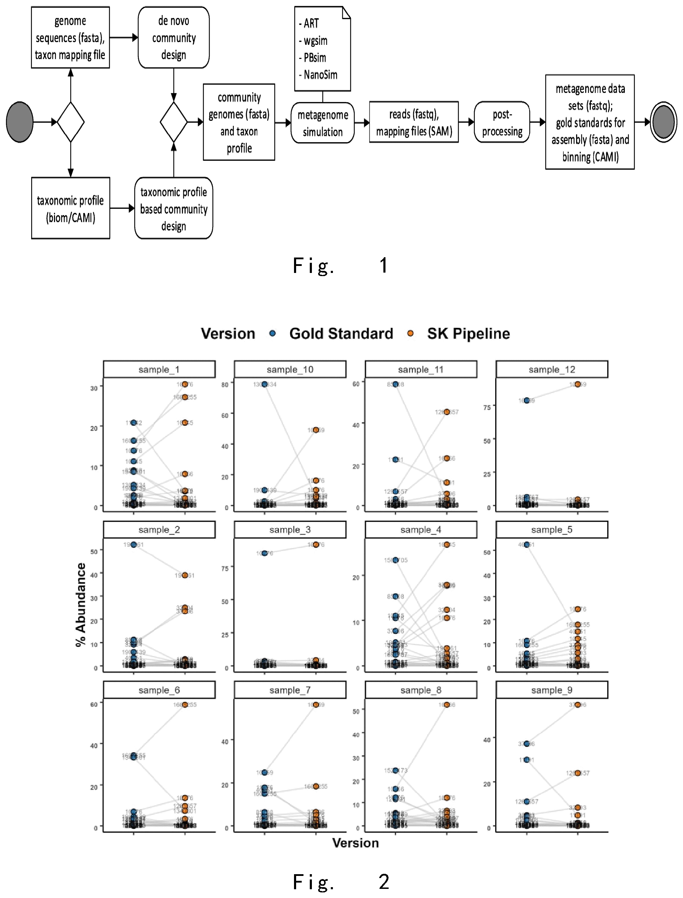

In one embodiment, the construction method of the present invention includes: 1) installing the CAMISIM tool; 2) downloading the reference genome of a specific microorganism; 3) referring to the CAMISIM tool teaching process, using the reference genome to perform standard data simulation (the process is shown in Figure 1); 4) performing data alignment on the simulated data through Kraken2 (reference), and removing the sequences aligned to non-target viral genomes.

In the process of constructing the digital standard of the present invention, good simulation sequence accuracy is achieved by using a specific amount of simulated data, simulated read length, and insert size. The read length used in the construction process of the present invention can be 50-500 bp, preferably 100-350 bp, more preferably 150-250 bp; the insert size can be 120 bp-400 bp, preferably 220 bp-320 bp, more preferably 270 bp-280 bp; the size of each sample in the simulated dataset can contain 1-10 G, preferably 3-5 G simulated reads. In a preferred embodiment, the construction process of the present invention adopts a read length of 150 bp, the insert size is set to 270 bp, and the data volume of each simulated sample is set to 5 G reads.

Virus detection is performed based on simulated data. Due to the sequence similarity, there is a probability of aligning to other viruses that are not the reference genome. Therefore, after simulating to obtain digital standards, the method of the present invention can further include a step of filtering the digital standards. Specifically, the data is filtered according to the base and sequence quality of the sequencing data of the digital standards; and sequence alignment software is used to align and identify the digital standards with a database built based on the reference genome, so as to remove sequences that align to non-target viral genomes. For the digital standards obtained in the present invention, sequences that are prone to being erroneously aligned to genomes not belonging to the reference viruses have been excluded. Using the standards of the present invention, there will basically be no cases of erroneous alignment.

### Main advantages of the present invention include:

1) For the present invention, the accuracy of the metagenomic bioinformatics process in the detection of specific viruses can be verified. It can help establish the accuracy and universality of using next-generation sequencing technology for biological product testing, which is conducive to promoting the application of next-generation sequencing in the field of biological products;
2) In the present invention, a set of metagenomic data containing 24 viruses from the Chinese Pharmacopoeia, including known virus species and virus abundance, has been developed. Therefore, it can be used to evaluate the sensitivity and specificity of metagenomic bioinformatics detection processes. It can also test the accuracy of process quantification.
3) There is a wide range of sources for the viruses selected in the present invention, including those from cattle, mice, humans, and pigs; and no or substantially no similar viral sequences are contained, which significantly reduces the interference of viral subtypes on the test results of digital standards; meanwhile, these viruses are all viruses of concern in the Chinese Pharmacopoeia, and the digital standards composed of them have extremely high clinical significance and application value.
4) In the present invention, the amount of simulation data, simulation read length, and insert size that are more favorable for the construction of digital standards are screened, and better simulation sequence accuracy is obtained.

The present invention is further elaborated below in conjunction with specific examples. It should be understood that these examples are only used to illustrate the present invention while not to limit the scope of the present invention. For the experimental methods without specifying specific conditions in the following examples, the conventional conditions are usually followed, such as those described in Sambrook et al., Molecular Cloning: A Laboratory Manual (New York: Cold Spring Harbor Laboratory Press, 1989), or the conditions suggested by the manufacturers. Unless otherwise specified, percentages and parts are by weight.

### Example 1 Construction of Metagenomic Bioinformatics Detection Standards

### 1.1 Installing the CAMISIM tool

Git was used to download version 71a1d86 from https://github.com/CAMI-challenge/CAMISIMm which was installed on the server according to the instructions.

### 1.2 Downloading the reference genome of specific microorganisms

The genomes of 24 key viruses of concern in the Chinese Pharmacopoeia (November 19, 2022 version) were downloaded from the NCBI GenBank database.

### 1.3 Metagenome Simulation Method 1: De novo Metagenome Simulation

### 1.3.1 Inputing Reference Genomes

For de novo metagenome simulation, all sequences shorter than 1000 base pairs were removed from the provided genome, and it was verified that the sequences contain only valid characters. The input genome can be a draft genome in fasta format, in which ambiguous DNA characters such as "RYWSMKHBVDN" in addition to the bases A, C, G, and T, may be contained. Among them, except for the "N" probability which is retained, all other non-base characters are removed.

### 1.3.2 Taxonomic Information

If the input taxonomic information was a taxonomic file in a CAMI or BIOM format, the taxa within it will be matched with closely related complete genomes (such as new genomes) from NCBI RefSeq or other reference files, so that the simulated metagenome (genomes and abundances thereof) can reflect the input profile as accurately as possible. This step was accomplished through the matching of scientific names in the input configuration file. Given the taxonomic names of Operational Taxonomic Units (OTUs) in a provided BIOM configuration file, NCBI taxonomic IDs can be inferred. Taxonomic IDs were also required for all reference genomes.

Next, the lineages between OTUs and genomes were compared, and OTUs were mapped to the genome with the smallest path between the OTU taxonomic ID and the genome taxonomic ID. The smallest path was calculated based on the de Bruijn graph. Since the smallest path can be very long, a threshold was set at the family level: an OTU will not be mapped to a genome not belonging to the same family, that is, for each mapped genome, it was ensured that they belong to at least the same family. Typically, this resulted in some OTUs failing to be mapped, either because their scientific names cannot be corresponding to NCBI taxonomic IDs, or because there are no complete genomes available for the taxonomic IDs. For unmapped OTUs, they can be assigned to a "random" genome in the reference set to increase complexity through additional options.

### 1.3.3 Community Design

For de novo community design, a specified number of sequences were selected from all input sequences (such as circular elements (i.e., a segment of sequences that can represent a certain microorganism) or genomes) according to certain criteria to generate a specific dataset. This specified number represented the number of virus sequence species to be generated through simulation. The novelty level was determined based on the similarity between the genome sequence combination and the currently known virus sequences. The random selection of genomes for a specific dataset was based on the membership thereof in novelty levels and OTUs: to increase taxonomic spread, for the total number of specified genomes to be included in a specific dataset, the selection algorithm attempted to extract an equal number of genomes from each novelty level (new strain, new species, new genus, new family, new order). If there were not enough genomes to reach the specified number of genomes in a category, more genomes will be extracted in equal amounts from other categories if possible. This operation was repeated until the specified number of genomes was extracted.

### 1.3.4 Addition of Extra Genomes

To increase the diversity at the strain level in a dataset, sgEvolver12 can be used to simulate many additional genomes, representing strains related to the reference genomes selected above.

To this end, a genome was randomly selected, and a strain number was drawn from a geometric distribution (p=0.3), which represented the number of related strains created for that specific genome. This process was repeated until the specified total number of strains to be generated was reached. For each genome, sgEvolver simulated 40 strains, with each strain having an increasing genetic distance from the previous one (in steps of 0.1%), reaching a total distance of up to 4%. The specified number of strains was randomly selected from these 40 strains and added to the genome set.

### 1.4 Metagenome Simulation Method 2: Dataset Simulation

For dataset simulation, each dataset required a genome and a circular element abundance distribution.

For a single sample dataset, the abundance distribution was created by sampling from a log-normal distribution. By default, the log mu of the log distribution was set to 1, and sigma was set to 2. A higher sigma results in a relatively flat normal distribution with a smaller peak. To reflect differential abundance experiments, two or more abundance samples can be independently drawn from the log-normal distribution using the same parameter settings.

For consecutive samples in a time series, the abundance of the next sample was calculated by sampling new values from a log-normal distribution, adding these values to the previous value, and then dividing by 2. The absolute abundance of circular elements can be set to 15 times the abundance of the microbial genome to replicate the high natural plasmid abundance. For each sample, the taxonomic profile of higher-rank taxa was calculated based on the genome abundance values and the taxonomic IDs thereof.

### 1.5 Reading Simulation

Based on the genome abundance distribution, genome size, and the specified output size of the metagenomic sample, the coverage in the simulated sample was calculated for each genome. Then, the selected read simulator generated reads using user-specified technologies and attributes (such as read length, insert size, or error rate, if applicable), and sampled each genome proportionally according to the specified abundance in the community. The read length was set to 150 bp, and the insert size was set to 270 bp. A second run of read simulation for the dataset can be performed using a different average insert size while maintaining the same genome abundance as before, to replicate the use of mixed technologies on the same sample. The size of each sample in each dataset was set to 5G reads.

The output of this step was for each sample in the dataset, as well as the FASTQ and BAM files, which specified the position of each read in the input sequence.

### 1.6 Assembly of Standards

SAMtools was used to perform gold-standard assembly for each sample and all samples in each dataset (merged gold standard), which respectively included each sequence position with a coverage of at least 1. Specifically, the SAM files obtained from read simulation specified the position of each read on the reference genome and were used with SAMtools to calculate the coverage of each locus. Then, the sequence in the reference genome was decomposed into gold-standard contig sequences at zero-coverage sites. The contig sequences were then labeled with a unique dataset and sample identification prefix as well as an incrementing index as a suffix. In the final step, the sequences in the simulated dataset were shuffled and anonymized using the unix command "shuf" to create the challenge dataset.

### Example 2: Building a metagenomic bioinformatics analysis workflow

Using the De novo simulation method, digital standards were constructed according to the method described in Example 1.

### 2.1 Building a self-developed metagenomic bioinformatics data analysis workflow

a) A common sequence quality control software, such as FastQC or FastP, was used to filter the data based on the base and sequence quality of the digital standard sequencing data.
b) A virus sequence alignment software (such as Kraken2, centrifuge, etc.) was downloaded and these 24 viral genomes were used to build a virus database.
c) The above software was used with preset parameters to perform virus sequence alignment and identification on the samples, and remove the sequences aligned to non-target viral genomes.

### 2.2 Accuracy Test of Bioinformatics Detection Methods

According to the types and abundances of viruses contained in each digital standard provided in this invention, the virus detection results were verified. The results are shown in Figure 2. The digital standards of the present invention can be used to judge the accuracy of bioinformatics detection methods.

### Comparison example

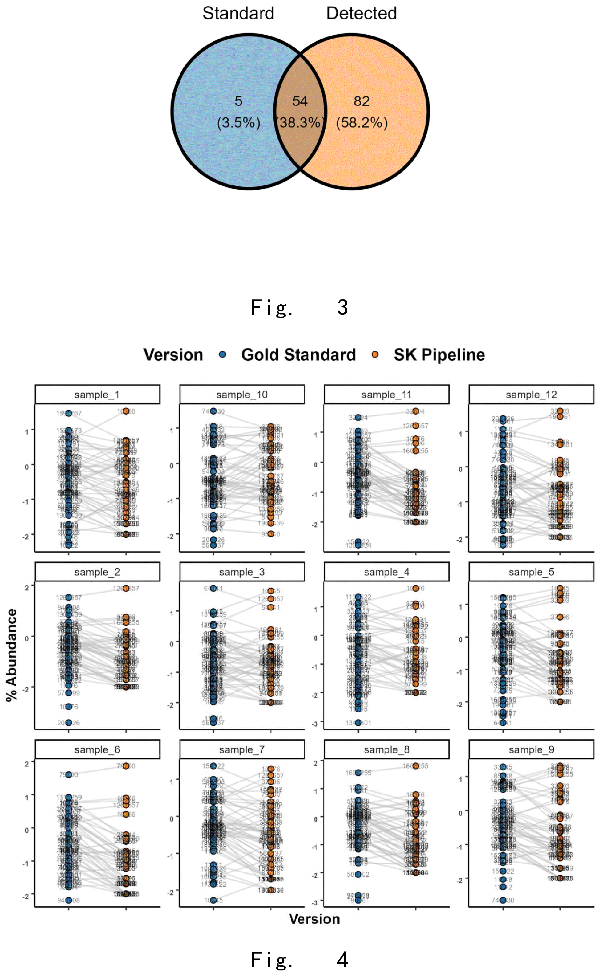

Due to the limitations of current sequence alignment methods and virus databases, it is extremely difficult to achieve completely accurate alignment of subtype viruses using alignment methods. Before attempting to use the 24 viruses in this study, the inventors prepared and tested digital standards using 59 viruses of concern in the Chinese Pharmacopoeia. The results showed that many false detection phenomena were produced by the standards constructed using these 59 viruses. As shown in Figure 3, the theoretically detected number of viruses should be around 59, but after detection using the bioinformatics workflow, a total of 136 virus species were detected. Meanwhile, the results in Figure 4 also showed that there were false detection and missed detection phenomena for some virus species in the standards.

After analysis, this phenomenon may be due to the interference of subtypes in these viruses. The genomic sequences in the digital standard are too similar to the sequences of other non-target viruses, thus resulting in false detection.

All documents mentioned in the present invention are cited as references in this application, just as each document is individually cited as a reference. In addition, it should be understood that after reading the above teaching contents of the present invention, those skilled in the art can make various changes or modifications to the present invention, and these equivalent forms also fall within the scope defined by the appended claims of this application.

## Claims

1. A method for preparing a digital standard for genomic bioinformation detection, which includes following steps:
(i) providing reference genomes of n microorganisms, where n is an integer selected from 10-24, preferably 15-24, and more preferably 20-24;
(ii) simulated-community design: generating a simulated community based on the reference genome and classification information;
(iii) reading simulation: using a read simulator to sample from each genome of the simulated community to generate simulated samples, and mixing the simulated samples to obtain a simulated dataset;
(iv) standard assembly: assembling the simulated dataset to obtain the digital standard.

2. The method for preparing a digital standard according to claim 1, wherein the microorganism is n microorganisms selected from the group consisting of:
*Betapolyomavirus macacae* (Simian Virus 40), *Bluetongue virus*, *Bovine polyomavirus 3*, *Human betaherpesvirus 6A* (Human betaherpesvirus-6), *Human betaherpesvirus 6B* (Human betaherpesvirus-6), *Fowl aviadenovirus A* (Exogenous Avian Adenovirus Type I), *Simian re*t*rovir*u*s 8* (Simian retrovirus), *Simian foamy virus*, *Human gammaherpesvirus 4* (Human Epstein-Barr virus) (Epstein-Barr virus), *Human polyomavirus 12* (Human polyomavirus), *Human circovirus VS6600022* (Human circovirus), *Human betaherpesvirus 5* (Human Cytomegalovirus), *Human immunodeficiency virus 1* (Human immunodeficiency virus), *Murine respirovirus* (Sendai Virus), *Murid betaherpesvirus 8* (Murine Salivary Gland Virus_Murine Cytomegalovirus), *Nipah henipavirus*, *Suid alphaherpesvirus 1* (Pseudorabies Virus), *Swinepox virus*, *Porcine reproductive and respiratory syndrome virus*, *Suid betaherpesvirus 2* (Porcine Cytomegalovirus), *Porcine circovirus 2*, *Human gammaherpesvirus 8* (Human Herpesvirus-8), *Bat rotavirus* (Mouse rotavirus), *Murid betaherpesvirus 1* (Murine Salivary Gland Virus_Murine Cytomegalovirus).

3. The method for preparing a digital standard according to claim 1, wherein the steps (ii) to (iv) are performed using CAMISIM software.

4. The method for preparing a digital standard according to claim 1, wherein the simulated dataset contains 10-500 simulated samples, preferably 50-200 simulated samples, and more preferably 100-150 simulated samples.

5. The method for preparing a digital standard according to claim 1, wherein the method further includes step (v) after step (iv): filtering the data according to the base and sequence quality of the sequencing data of the digital standard; and using sequence alignment software to compare and identify the digital standard with a database built based on the reference genome, and remove the sequences that are aligned to non-target viral genomes.

6. A digital standard for metagenomic bioinformatics detection, wherein the digital standard comprises p samples, each sample contains genomic sequences from reference genomes of n different microorganisms,
the p samples have different genomic abundances,
p is an integer selected from 10-500, preferably 50-200, and more preferably 100-150, and
n is an integer selected from 10-24, preferably 15-24, and more preferably 20-24.

7. The digital standard according to claim 6, wherein the n microorganisms consist of: *Betapolyomavirus macacae* (Simian Virus 40), *Bluetongue virus*, *Bovine polyomavirus 3*, *Human betaherpesvirus 6A* (Human betaherpesvirus-6), *Human betaherpesvirus 6B* (Human betaherpesvirus-6), *Fowl aviadenovirus A* (Exogenous Avian Adenovirus Type I), *Simian re*t*rovir*u*s 8* (Simian retrovirus), *Simian foamy virus*, *Human gammaherpesvirus 4* (Human Epstein-Barr virus) (Epstein-Barr virus), *Human polyomavirus 12* (Human polyomavirus), *Human circovirus VS6600022* (Human circovirus), *Human betaherpesvirus 5* (Human Cytomegalovirus), *Human immunodeficiency virus 1* (Human immunodeficiency virus), *Murine respirovirus* (Sendai Virus), *Murid betaherpesvirus 8* (Murine Salivary Gland Virus_Murine Cytomegalovirus), *Nipah henipavirus*, *Suid alphaherpesvirus 1* (Pseudorabies Virus), *Swinepox virus*, *Porcine reproductive and respiratory syndrome virus*, *Suid betaherpesvirus 2* (Porcine Cytomegalovirus), *Porcine circovirus 2*, *Human gammaherpesvirus 8* (Human Herpesvirus-8), *Bat rotavirus* (Mouse rotavirus), *Murid betaherpesvirus 1* (Murine Salivary Gland Virus_Murine Cytomegalovirus).

8. The digital standard according to claim 6, wherein the digital standard is filtered, and the filtering refers to align and identify the digital standard with a database built based on the reference genome by using sequence alignment software, and removing sequences that align to non-target viral genomes.

9. The digital standard according to claim 6, wherein the digital standard is constructed by the method according to claim 1.

10. A device for preparing a digital standard for metagenomic bioinformatics detection, and the device includes:
1) an input module, which is used to input reference genomes of n microorganisms, wherein n is an integer selected from 10-24, preferably 15-24, and more preferably 20-24;
2) a simulation module, which is used to:
generate a simulated community based on the reference genomes and classification information;
sample from each genome of the simulated community by using a read simulator to generate simulated samples, and mix the simulated samples to obtain a simulated dataset;
assemble the simulated dataset to obtain the digital standard; and
3) an output module, which is used to output the digital standard.

11. The device according to claim 10, wherein the n microorganisms consist of: *Betapolyomavirus macacae* (Simian Virus 40), *Bluetongue virus*, *Bovine polyomavirus 3*, *Human betaherpesvirus 6A* (Human betaherpesvirus-6), *Human betaherpesvirus 6B* (Human betaherpesvirus-6), *Fowl aviadenovirus A* (Exogenous Avian Adenovirus Type I), *Simian re*t*rovir*u*s 8* (Simian retrovirus), *Simian foamy virus*, *Human gammaherpesvirus 4* (Human Epstein-Barr virus) (Epstein-Barr virus), *Human polyomavirus 12* (Human polyomavirus), *Human circovirus VS6600022* (Human circovirus), *Human betaherpesvirus 5* (Human Cytomegalovirus), *Human immunodeficiency virus 1* (Human immunodeficiency virus), *Murine respirovirus* (Sendai Virus), *Murid betaherpesvirus 8* (Murine Salivary Gland Virus_Murine Cytomegalovirus), *Nipah henipavirus*, *Suid alphaherpesvirus 1* (Pseudorabies Virus), *Swinepox virus*, *Porcine reproductive and respiratory syndrome virus*, *Suid betaherpesvirus 2* (Porcine Cytomegalovirus), *Porcine circovirus 2*, *Human gammaherpesvirus 8* (Human Herpesvirus-8), *Bat rotavirus* (Mouse rotavirus), *Murid betaherpesvirus 1* (Murine Salivary Gland Virus_Murine Cytomegalovirus).

12. A method for verifying the accuracy of metagenomic bioinformatics detection, which includes following steps:
I) providing a metagenomic bioinformatics detection method to be tested;
II) using the detection method to detect each sample in the digital standard according to any one of claims 6-10, so as to obtain the detection results of the detection method;
III) comparing the detection results of the detection method with the reference genome species and corresponding abundance information of each sample in the digital standard, so as to judge the accuracy of the detection method.

13. A device for verifying the accuracy of a metagenomic bioinformatics detection method, and the device includes:
I) an input module, which is used for inputting the metagenomic bioinformatics detection method to be tested;
II) a calculation module, which uses the detection method to detect the digital standard according to any one of claims 6-10, so as to obtain the detection result of the method to be tested;
III) a comparison module, which compares the detection result of the detection method with the reference genome species and corresponding abundance information of each sample in the digital standard, so as to judge the accuracy of the bioinformatics detection method;
IV) an output module, which is used for outputting the accuracy judgment result of the bioinformatics detection method.
